# FASCICULE DE BREVET EUROPEEN

(11) **EP 3 204 063 B1**
(45) Date de publication et mention de la délivrance du brevet: **01.12.2021**
(21) Numéro de dépôt: 15780817.1
(22) Date de dépôt: 09.10.2015
(51) Int. Cl.: A61M 60/50, A61M 60/871

(54) **DISPOSITIF DE GESTION DU FONCTIONNEMENT D'UNE PROTHESE CARDIAQUE**
VORRICHTUNG ZUR STEUERUNG EINER HERZPROTHESE
CONTROL DEVICE FOR CARDIAC PROSTHESIS

(30) Priorité: 10.10.2014 FR 1459759
(43) Date de publication de la demande: 16.08.2017
(73) Titulaire: Avantix, 13794 Aix-en-Provence cedex 3 (FR)
(72) Inventeur: GRANDY, Patrick, F-13340 Rognac (FR)
(74) Mandataire: Cabinet Camus Lebkiri
(86) Numéro de dépôt international: PCT/EP2015/073481
(87) Numéro de publication internationale: WO 2016/055652

(56) Documents cités:
- EP-A1- 2 255 839
- WO-A1-2012/012552
- WO-A1-2013/173643

## Description

### DOMAINE TECHNIQUE

La présente invention se rapporte à un dispositif de gestion du fonctionnement d'une prothèse cardiaque. Plus particulièrement, le dispositif de gestion du fonctionnement d'une prothèse cardiaque selon l'invention vise à surveiller et piloter l'alimentation électrique d'une prothèse cardiaque. Il vise également à surveiller le fonctionnement de la prothèse cardiaque de manière à pouvoir agir sur la prothèse ou créer un avertissement lors d'un dysfonctionnement de cette dernière.

### ETAT DE LA TECHNIQUE ANTERIEUR

Habituellement, lorsqu'une personne souffre d'une déficience cardiaque importante, il arrive qu'une greffe cardiaque soit la seule voie de guérison envisageable. Dans ce cas de figure, le cœur déficient est remplacé par un cœur sain prélevé sur un donneur. Face au nombre limité de donneur, une prothèse cardiaque implantable et autonome visant à reproduire fidèlement le fonctionnement du cœur naturel a été développée. L'énergie électrique nécessaire au fonctionnement de la prothèse cardiaque peut être fournie de deux manières différentes.

Une première manière consiste à fournir l'énergie électrique nécessaire au fonctionnement de la prothèse cardiaque via une batterie portative que le patient peut porter sur lui. La prothèse cardiaque est alors reliée à la batterie portative via un fil passant par une micro-perforation cutanée. Ainsi, la batterie portative externe assure l'alimentation électrique de l'électronique de la prothèse cardiaque via ce fil.

Une seconde manière consiste à fournir l'énergie électrique nécessaire au fonctionnement de la prothèse cardiaque via une console hospitalière. Ce type d'alimentation est généralement utilisé en période post-opératoire.

Chaque alimentation est pilotée par un dispositif distinct fonctionnant de façon indépendante. Plus particulièrement, l'alimentation portative est pilotée par un dispositif de pilotage portatif tandis que l'alimentation fournie par la console hospitalière est pilotée par la console hospitalière. Ces deux dispositifs de pilotage d'alimentation sont évidements peu pratique et couteux.

### EXPOSE DE L'INVENTION

L'invention a donc pour objectif de remédier aux inconvénients de l'état de la technique. Dans ce contexte, la présente invention a pour but de fournir un dispositif de gestion du fonctionnement d'une prothèse cardiaque qui soit sécuritaire pour la personne munie d'une telle prothèse cardiaque et peu couteux.

A cette fin, l'invention porte sur un dispositif de gestion du fonctionnement d'une prothèse cardiaque, le dispositif de gestion du fonctionnement d'une prothèse cardiaque comportant une voie de gestion, la voie de gestion comportant :
- des moyens de gestion construits et agencés pour surveiller et commander l'alimentation électrique d'une prothèse cardiaque,
- des premiers moyens d'isolation construits et agencés pour isoler électriquement la prothèse cardiaque de l'alimentation électrique et des moyens de gestion construits et agencés pour surveiller et commander l'alimentation électrique,
- une interface de communication avec la prothèse cardiaque, et
- un isolateur de communication disposé entre les moyens de gestion et l'interface de communication.

Grâce à l'invention, un même dispositif est adapté pour surveiller et commander l'alimentation électrique d'une prothèse cardiaque. Par ailleurs, le dispositif selon l'invention est muni de premiers moyens d'isolation électrique de la prothèse cardiaque avec l'alimentation électrique, ce qui permet d'assurer une sécurité de fonctionnement de la prothèse cardiaque, et par voie de conséquence la sécurité du porteur de prothèse cardiaque.

Le dispositif de gestion du fonctionnement d'une prothèse cardiaque selon l'invention peut également présenter une ou plusieurs des caractéristiques ci-après, considérées individuellement ou selon toutes les combinaisons techniquement réalisables.

Dans une mise en œuvre non limitative, l'alimentation électrique est formée par une alimentation électrique portative et/ou une alimentation électrique fixe.

Dans une mise en œuvre non limitative, les premiers moyens d'isolation sont construits et agencés pour isoler électriquement la prothèse cardiaque de l'alimentation électrique fixe.

Dans une mise en œuvre non limitative, les premiers moyens d'isolation sont construits et agencés pour isoler électriquement la prothèse cardiaque de l'alimentation électrique portative.

Dans une mise en œuvre non limitative, les premiers moyens d'isolation sont conformes à la norme NF-EN 60601-1.

Dans une mise en œuvre non limitative, l'interface de communication est formée par une interface de type CAN ou de type RS232.

Dans une mise en œuvre non limitative, la voie de gestion comporte des moyens de mesure construits et agencés pour mesurer une pression atmosphérique.

Dans une mise en œuvre non limitative, la voie de gestion comporte des moyens d'avertissement construits et agencés pour communiquer avec les moyens de gestion de sorte à avertir un utilisateur lorsqu'un défaut de fonctionnement de l'alimentation électrique, de la voie de gestion, d'une deuxième voie de gestion et/ou de la prothèse cardiaque est détecté par les moyens de gestion.

Dans une mise en œuvre non limitative, la voie de gestion comporte au moins une interface externe construite et agencée pour permettre des communications entre les moyens de gestion et un dispositif externe.

Dans une mise en œuvre non limitative, le dispositif de gestion du fonctionnement d'une prothèse cardiaque comporte en outre une deuxième voie de gestion, la deuxième voie de gestion étant conforme à la première voie de gestion selon l'invention.

Dans une mise en œuvre non limitative, la première voie de gestion et la deuxième voie de gestion sont montées en parallèle, le dispositif de gestion du fonctionnement comportant en outre des deuxièmes moyens d'isolation de la première voie de gestion et de la deuxième voie de gestion.

### BREVE DESCRIPTION DES FIGURES

D'autres caractéristiques et avantages de l'invention ressortiront clairement de la description qui en est donnée ci-dessous, à titre indicatif et nullement limitatif, en référence aux figures annexées, parmi lesquelles :
- la figure 1 illustre, de façon schématique, un premier exemple de réalisation d'un dispositif de gestion du fonctionnement d'une prothèse cardiaque conforme à l'invention,
- la figure 2 illustre, de façon schématique, un deuxième exemple de réalisation d'un dispositif de gestion du fonctionnement d'une prothèse cardiaque conforme à l'invention,
- la figure 3 illustre, de façon schématique, un troisième exemple de réalisation d'un dispositif de gestion du fonctionnement d'une prothèse cardiaque conforme à l'invention.

Pour des raisons de clarté, seuls les éléments utiles pour la compréhension de l'invention ont été représentés et ceci, sans respect de l'échelle et de manière schématique. En outre, les éléments similaires situés sur différentes figures comportent des références identiques.

### DESCRIPTION DETAILLEE D'AU MOINS UN MODE DE REALISATION DE L'INVENTION

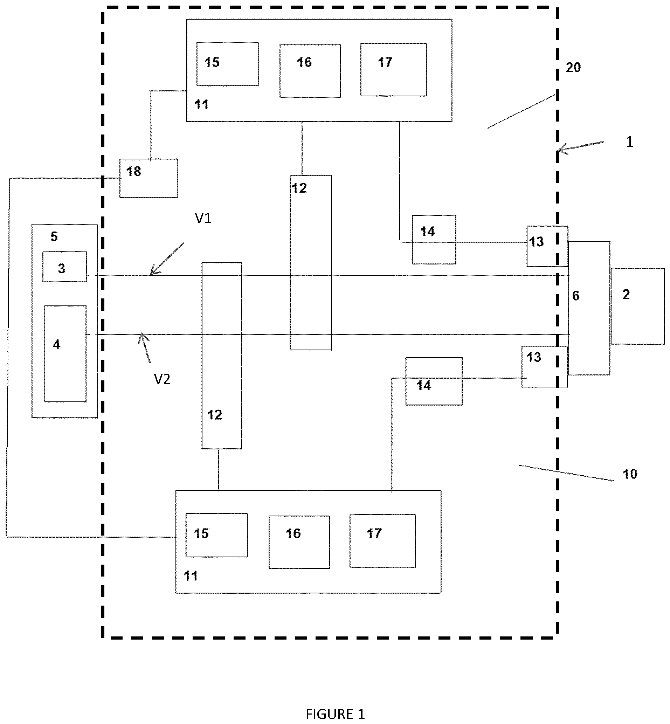

La figure 1 illustre un exemple de réalisation d'un dispositif de gestion 1 du fonctionnement d'une prothèse cardiaque 2 conforme à l'invention.

Plus particulièrement, une prothèse cardiaque 2 de ce type présente un dispositif interne non représenté. Un tel dispositif interne est constitué de capteurs situés sur la prothèse cardiaque 2 reliés à un micro-processeur localisé sous la coque de la prothèse cardiaque 2. Pour assurer une régulation cardiaque répondant aux besoins variables de la personne portant cette prothèse, les capteurs enregistrent des données de pression artérielle et de position (repos et efforts physique). Le microprocesseur reçoit ces informations et les traite en permanence. Véritable ordinateur de bord, il assure alors l'adéquation du rythme cardiaque au besoin du corps, notamment en activant plus ou moins rapidement des motopompes que comporte la prothèse 2.

Le dispositif de gestion 1 du fonctionnement de la prothèse cardiaque 2 selon l'invention est formé par un boitier pouvant être fixe ou porté sur le patient. Le dispositif de gestion 1 fournit des informations au patient et relaye des données de télédiagnostics vers l'hôpital afin d'assurer le suivi à distance du porteur de la prothèse cardiaque 2. Ce service de monitoring 24h/24 permet le suivi des données internes liées à la prothèse cardiaque 2 ainsi que des données physiologiques du patient.

D'une manière générale, pour assurer la sécurité du patient, le dispositif de gestion 1 présente deux voies d'alimentation électrique V1 et V2 totalement indépendantes l'une de l'autre au cas où l'une d'entre elles serait défectueuse.

Lorsque le dispositif de gestion 1 est fixe (autrement dit connecté à une console hospitalière) ou portable et est en fonctionnement dit autonome, deux alimentations 5 peuvent être utilisées, à savoir une batterie de la console hospitalière 4 et une batterie portative 3. Ainsi, l'énergie électrique nécessaire au fonctionnement de la prothèse cardiaque 2 est fournie par une batterie portative 3 et/ou par une batterie de la console hospitalière 4. La prothèse cardiaque 2 est connectée à la batterie portative 3, à la console hospitalière 4 et au dispositif de gestion 1 via une interface de connexion 6 accessible via une micro-perforation percutanée du porteur de la prothèse cardiaque 2. En conséquence, cette interface de connexion 6 permet de brancher l'alimentation électrique 5 et, aussi le dispositif de gestion 1, lequel véhicule les informations entre l'électronique embarquée par la prothèse cardiaque 2 et lui-même de manière à pouvoir assurer la surveillance et le pilotage de la prothèse cardiaque 2.

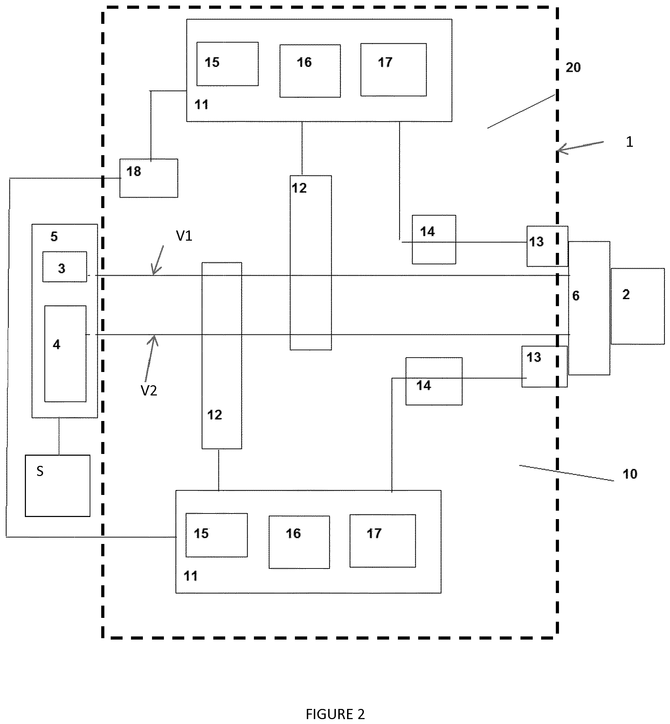

Dans une mise en œuvre non limitative illustrée sur la figure 2, le dispositif de gestion 1 peut en outre être connecté au secteur S, dans ce cas les deux batteries 3 et 4 sont présentes mais l'énergie électrique est fournie par une alimentation réalisée à partir du secteur S. Les batteries 3 et/ou 4 ne servent qu'en secours lors d'une coupure du secteur S. Le secteur S peut également permettre de recharger les batteries notamment celle de la batterie portative 3.

Le dispositif de gestion 1 illustré sur la figure 1 et sur la figure 2 comporte une première voie de gestion 10 et une deuxième voie de gestion 20.

La première voie de gestion 10 comporte des moyens de gestion 11 construits et agencés pour surveiller et commander l'alimentation électrique 5 de la prothèse cardiaque 2, l'alimentation électrique 5 étant formée par une alimentation électrique portative 3 et une alimentation électrique fixe 4 d'une console hospitalière.

La première voie de gestion 10 comporte également des premiers moyens d'isolation 12 construits et agencés pour isoler électriquement la prothèse cardiaque 2 de l'alimentation électrique 5. Ainsi, lorsque la prothèse cardiaque 2 est branchée sur une alimentation électrique 5, par exemple en période post-opératoire, alors le dispositif de gestion 1 permet, par exemple, de protéger la prothèse cardiaque 2 contre une surintensité. Par ailleurs, les premiers moyens d'isolation 12 sont également construits et agencés pour isoler électriquement la prothèse cardiaque 2 des moyens de gestion 11. Par exemple, les premiers moyens d'isolation 12 sont conformes à la norme NF-EN 60601 -1.

Il convient de noter que l'alimentation électrique portative 3 peut être de type lithium ou de type à pile à hydrogène.

La première voie de gestion 10 comporte en outre une interface de communication 13 avec la prothèse cardiaque 2. Cette interface de communication 13 peut, par exemple, être de type CAN ou de type RS232. Dans le cas d'une interface de communication 13 de type RS232, la première voie de gestion 10 comporte un isolateur de communication 14 disposé entre les moyens de gestion 11 et l'interface de communication 13 de type RS232. Dans le cas d'une interface de communication 13 de type CAN, la première voie de gestion 10 comporte un isolateur de communication 14 disposé entre les moyens de gestion 11 et l'interface de communication 13 de type CAN.

Par ailleurs, la première voie de gestion 10 comporte des moyens de mesure 15 construits et agencés pour mesurer la pression atmosphérique. Ces moyens de mesure 15 peuvent être formé par un capteur de pression communiquant avec la prothèse cardiaque 2 via l'interface de communication 13. Ces moyens de mesure 15 sont en outre construits et agencés pour communiquer avec les moyens de gestion 11.

La première voie de gestion 10 comporte des moyens d'avertissement 16 construits et agencés pour communiquer avec les moyens de gestion 11 de sorte à créer un avertissement. Ces moyens d'avertissement 16 peuvent être formés par un voyant lumineux et/ou par un buzzer.

Les moyens de gestion 11 sont adaptés et construits pour détecter un disfonctionnement de l'alimentation électrique 5, un disfonctionnement de la communication avec la prothèse cardiaque 13, un disfonctionnement d'une deuxième voie de gestion 20 et de manière plus générale un disfonctionnement de la prothèse cardiaque 2. Ainsi, lorsqu'un disfonctionnement de l'alimentation électrique 5 se produit et est détecté par les moyens de gestion 11, alors les moyens de gestion 11 peuvent agir sur les premiers moyens d'isolation 12 pour protéger la prothèse cardiaque 2. En outre, lorsqu'un disfonctionnement de la prothèse cardiaque 2 est détecté par les moyens de gestion 11, ces derniers peuvent déclencher une alarme visant à informer un praticien et/ou le porteur de la prothèse cardiaque 2.

La première voie de gestion 10 comporte en outre au moins une interface 17 construite et agencée pour permettre des communications entre les moyens de gestion 11 et un dispositif externe. Par exemple, cette interface 17 peut être de type Ethernet, USB, Wifi ou toute autre interface permettant de connecter, par voie filaire ou sans fil, les moyens de gestion 11 avec un dispositif externe.

Le dispositif de gestion 1 comporte également une deuxième voie de gestion 20, la deuxième voie de gestion 20 est identique à la première voie de gestion 10 excepté pour l'interface de communication 13 avec la prothèse cardiaque 2 qui peut être différente. Par conséquent, dans l'exemple illustré sur la figure 1, la deuxième voie de gestion 20 comporte :
- des moyens de gestion 11 construits et agencés pour surveiller et commander l'alimentation électrique 5 de la prothèse cardiaque 2,
- des premiers moyens d'isolation 12 construits et agencés pour isoler électriquement la prothèse cardiaque 2 de l'alimentation électrique 5 et des moyens de gestion 11,
- une interface de communication 13 avec la prothèse cardiaque 2, dans le cas d'une interface de communication 13 de type RS232 13, la deuxième voie de communication 20 comporte en outre un isolateur de communication 14 disposé entre les moyens de gestion 11 et l'interface de communication 13 de type RS232, dans le cas d'une interface de communication 13 de type CAN, la deuxième voie de gestion 20 comporte en outre un isolateur de communication 14 disposé entre les moyens de gestion 11 et l'interface de communication 13 de type CAN,
- des moyens de mesure 15 construits et agencés pour mesurer la pression atmosphérique,
- des moyens d'avertissement 16 construits et agencés pour communiquer avec les moyens de gestion 11 de sorte à créer un avertissement lors d'un dysfonctionnement de l'alimentation électrique 5, un disfonctionnement de la communication avec la prothèse cardiaque 13, un disfonctionnement de la première voie de gestion 10 et/ou un disfonctionnement de la prothèse cardiaque 2,
- au moins une interface 17 construite et agencée pour permettre des communications entre les moyens de gestion 11 et un dispositif externe.

Il convient de noter que la première voie de gestion 10 et la deuxième voie de gestion 20 sont montées en parallèle. De façon à protéger la première voie de gestion 10 de la deuxième voie de gestion 20, et inversement, le dispositif de gestion 1 conforme à l'invention comporte des deuxièmes moyens d'isolation 18 de la première voie de gestion 10 avec la deuxième voie de gestion 20.

En d'autres termes, le dispositif de gestion 1 est formé de deux voies de gestion 10 et 20 fonctionnant de façon totalement indépendante. Chacune des deux voies permettant notamment de gérer :
- les sources d'énergie électrique afin d'assurer que l'énergie électrique soit réellement fournie à la prothèse cardiaque 2, et ce de façon certaine,
- une liaison de communication avec la prothèse cardiaque 2, par exemple une liaison de type CAN sur la première voie 10 et une liaison de type RS232 sur la deuxième voie 20,
- un avertissement en cas de défaillance de l'alimentation électrique 5 et/ou de la prothèse cardiaque 2 et/ou des voies de gestion 10 et 20 au moyen d'une alarme sonore et/ou visuelle,
- le dialogue avec l'autre voie de gestion de manière à détecter un disfonctionnement de l'autre voie.

Par ailleurs, dans l'exemple illustré, les moyens de gestion 11 sont alimentés électriquement via l'alimentation électrique 5.

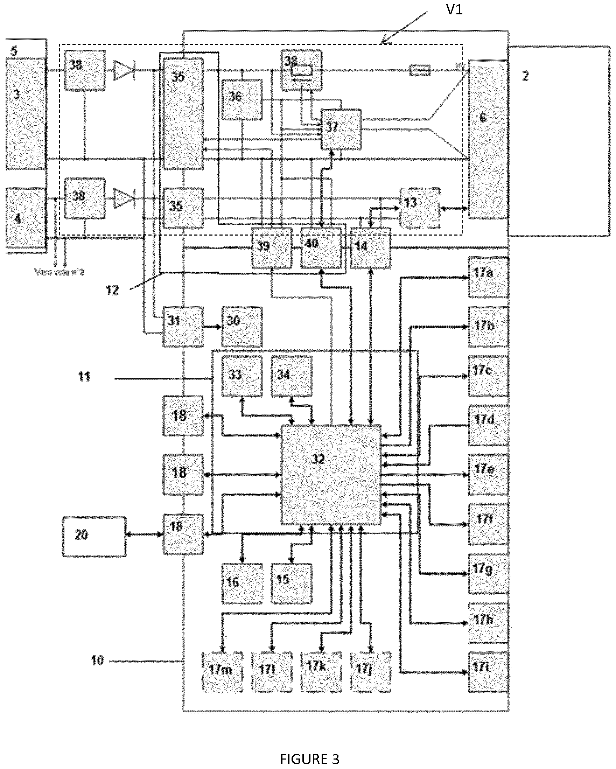

La figure 3 illustre un exemple de réalisation d'une voie de gestion 10 du dispositif de gestion 1 conforme à l'invention. Une seule voie de gestion est représentée par soucis de clarté.

Dans cet exemple non limitatif, la voie de gestion 10 comporte des moyens de gestion 11 construits et agencés pour surveiller et commander l'alimentation électrique 5 de la prothèse cardiaque 2. Dans cet exemple, l'alimentation électrique 5 est formée par une alimentation électrique portative 3 et une alimentation électrique fixe 4. Ces deux alimentations électriques sont connectées à une interface de connexion 6 connectée à la prothèse cardiaque 2 via une première voie d'alimentation électrique V1. L'agencement de cette voie d'alimentation électrique V1 est donné ici à titre d'exemple et peut être différente. En outre, les différentes fonctionnalités des différents modules représentés de la voie d'alimentation électrique V1 seront décrites ultérieurement.

Par ailleurs, l'alimentation électrique 5 est utilisée pour alimenter en énergie l'alimentation 30 des moyens de gestion 11. Pour ce faire, un convertisseur 31 de type DC/DC est disposé entre l'alimentation électrique 5 et l'alimentation 30 des moyens de gestion 11. L'alimentation électrique des moyens de gestion 11 peut être de l'ordre de 5V. Le convertisseur 31 de type DC/DC peut être conforme à la norme NF-EN 60601-1.

A titre illustratif, les moyens de gestion 11 peuvent être formés par :
- un processeur 32, par exemple de type I.MX6,
- une mémoire dynamique 33, cette mémoire dynamique peut, par exemple, être formée par quatre boitiers de type DDR3L de 256 MO à 1 GO chacun, et
- une mémoire de masse 34, par exemple de type eMMC de 8 GO à 64 GO.

Cette mise en œuvre permet d'implémenter un maximum de fonction dans un minimum de composants. Grâce à cette particularité, chacun des moyens de gestion 11 consomme mois de 5W. De ce fait, la majorité de l'alimentation électrique 5 est fournie à la prothèse cardiaque 2.

La voie de gestion 10 comporte non seulement des moyens de gestion 11 construits et agencés pour surveiller et commander l'alimentation électrique 5 de la prothèse cardiaque 2, mais également des premiers moyens d'isolation 12 construits et agencés pour isoler électriquement la prothèse cardiaque 2 des moyens de gestions 11, ces moyens de gestions 11 pouvant être reliés à un potentiel fixe via une ou des liaisons externes. Les premiers moyens d'isolation 12 sont également construits et agencés pour isoler électriquement la prothèse cardiaque 2 de l'alimentation électrique 5.

De manière générale, les moyens de gestion 11 et les premiers moyens d'isolation 12 communiquent de manière à pouvoir :
- stopper le fonctionnement des boitiers convertisseurs DC/DC isolés 35, cette fonction peut être utilisée pour des besoins d'autotest ou pour des problèmes de sécurité (exemple : lorsque les moyens de gestion 11 détectent que la prothèse cardiaque 2 est en court-circuit pendant un temps déterminé) ; ces boitiers convertisseurs DC/DC isolés 35 permettent d'isoler électriquement la prothèse cardiaque 2 de l'alimentation électrique 5 si nécessaire,
- surveiller les tensions d'alimentation pour détecter les défauts de la prothèse cardiaque 2,
- ajuster la tension, via les boitiers convertisseurs DC/DC isolés 35 pilotés par le contrôleur 37 alimenté par le régulateur de tension 36, pour permettre un équilibre du courant fourni par chaque voie d'alimentation électrique,
- mesurer le courant, via le contrôleur 37, fourni à la prothèse cardiaque 2 (cette information permet de confirmer que l'alimentation est réellement fournie à la prothèse cardiaque 2), détecter, via le contrôleur 37, des surintensités sur le courant fourni à la prothèse cardiaque 2, ou
- commander des tests des boitiers de protection 38 contre les surtensions ou surintensité, par exemple ces tests peuvent consister à abaisser le seuil de détection pour vérifier son déclenchement.

Toutes les communications entre les moyens de gestion 11 et les fonctions d'alimentation de la prothèse cardiaque 2 sont réalisées au travers des premiers moyens d'isolation 12 et respectent par exemple la norme NF-EN 60601-1 avec des courants de fuite inférieurs à la catégorie CF (courants de fuite de 0,22µA typiquement sous 264V 60Hhz par isolateur).

Ces premiers moyens d'isolation 12 peuvent comporter non seulement les boitiers convertisseurs DC/DC isolés 35 mais également un isolateur numérique 39 et un isolateur de type I2C 40.

Les boitiers convertisseurs DC/DC isolés 35 permettent notamment d'isoler électriquement la prothèse cardiaque 2 de l'alimentation électrique 5.

L'isolateur numérique 39 et l'isolateur de type I2C 40 permettent notamment d'isoler électriquement la prothèse cardiaque 2 des moyens de gestion 11.

Par ailleurs, la voie 10 de gestion comporte une interface de communication 13 avec la prothèse cardiaque 2. Cette interface de communication 13 peut être de type CAN ou de type RS232. Dans le cas d'une interface de communication 13 de type RS232, un isolateur de communication 14 est disposé entre les moyens de gestion 11 et l'interface de communication 13 de type RS232.

De manière générale, la communication entre les moyens de gestion 11 et la prothèse cardiaque 2 peut être effectuée :
- sur une voie de gestion (par exemple la première voie de gestion 10), via une interface de communication de type RS232, après passage au travers d'un isolateur de communication 14 et mise à niveau par un driver RS232 13,
- sur l'autre voie de gestion (la deuxième voie de gestion 20), via une interface de communication de type CAN au travers d'un isolateur de communication14 et sans nécessiter de driver CAN.

Il est entendu que la première voie de gestion 10 peut comporter une interface de communication 13 de type CAN ou RS232 et la deuxième voie de gestion 20 peut comporter une interface de communication 13 de type CAN ou RS232.

En outre, la voie de gestion 10 comporte des moyens de mesure construits et agencés pour mesurer la pression atmosphérique 15. Ces moyens de mesure de pression atmosphérique 15 peuvent être formés par un capteur de pression. Ce dernier peut en outre être soudé sur la carte électronique du processeur 32 que comportent les moyens de gestion 11.

La voie de gestion 10 comporte également des moyens d'avertissement 16 construits et agencés pour communiquer avec les moyens de gestion 11. Ces moyens d'avertissement 16 permettent d'avertir le porteur de la prothèse cardiaque 2 et/ou un hôpital dès lors qu'un défaut de fonctionnement de l'alimentation électrique 5 et/ou de la prothèse cardiaque 2 et/ou des voies de gestion 10 et 20 est détecté par les moyens de gestion 11. Ces moyens d'avertissement 16 peuvent, par exemple, être formés par un buzzer communiquant avec les moyens de gestion 11.

En outre, la voie de gestion 10 comporte des interfaces externes 17 construites et agencées pour permettre des communications entre les moyens de gestion 11 et des dispositifs internes et/ou externes.

Dans l'exemple illustré, les interfaces externes 17 sont formées par :
- Une interface de type RS232 17a permettant par exemple de connecter un capteur de pression,
- Une interface vidéo 17b de type LVDS ou parallèle RGB permettant de connecter un écran que comporte les moyens de gestion 11,
- Une première interface 17c de type USB 2 ou SPI permettant de connecter un écran tactile que comporte les moyens de gestion 11,
- Une interface clavier matriciel 17d permettant de connecter un clavier que comportent les moyens de gestion 11,
- Une interface 17e permettant de connecter des voyants lumineux pouvant être utilisé pour générer une alerte ;
- Une interface vidéo de type HDMI 17f permettant de connecter un écran externe,
- Une deuxième interface 17g de type USB 2 permettant de connecter un écran tactile externe,
- Une troisième interface 17h de type USB 2 permettant de connecter une liaison externe,
- Une interface Gigabit Ethernet 17i permettant de connecter un dispositif externe,
- Une interface 17j permettant de connecter un disque de type SSD, ce disque de type SSD peut être embarqué par les moyens de gestion 11 ou être extérieur à celui-ci,
- Une interface 17k permettant de connecter un modem de type WIFI,
- Une interface 17I permettant de connecter un modem de type Bluetooth,
- Une interface 17m permettant de connecter un modem de type GSM.

Il convient de noter que ces interfaces 17 sont listées qu'à titre indicatif. L'homme du métier est capable d'en supprimer ou en ajouter sans pour autant sortir du cadre de l'invention.

Par ailleurs, le dispositif de gestion 1 du fonctionnement d'une prothèse cardiaque selon l'invention comporte une deuxième voie de gestion 20. Cette deuxième voie de gestion 20 est identique à la première voie de gestion 10. En outre, la première voie de gestion 10 et la deuxième voie 20 de gestion sont montées en parallèle. Ces deux voies de gestion 10 et 20 sont isolées entre elles via des deuxièmes moyens d'isolation 18. Ces deuxièmes moyens d'isolation 18 peuvent être formés par un isolateur de type numérique. Plus particulièrement les deuxièmes moyens d'isolation 18 peuvent être formés par des isolateurs I2C (compatible SMBus). Ces moyens d'isolation 18 permettent de dialoguer avec l'alimentation électrique 5 pour connaitre, notamment :
- La tension,
- Le courant débité,
- La capacité restante de la batterie,
- La température de la batterie,
- L'état de la batterie.

Ces informations sont essentielles pour la sécurité du patient lorsque le dispositif de gestion 1 est en mode autonome. En outre, ces deuxièmes moyens d'isolation 18 garantissent une totale indépendance des deux voies de gestion même en cas de défaut d'une des deux voies de gestion.

## Revendications

1. Dispositif de gestion (1) du fonctionnement d'une prothèse cardiaque (2), ledit dispositif de gestion (1) du fonctionnement d'une prothèse cardiaque (2) comportant une voie de gestion (10), ladite voie de gestion (10) comportant :
- une alimentation électrique (5) formée par une alimentation électrique portative (3) et une alimentation électrique fixe (4),
- des moyens de gestion (11) construits et agencés pour surveiller et commander l'alimentation électrique (5) d'une prothèse cardiaque (2),
- des premiers moyens d'isolation (12) construits et agencés pour isoler électriquement la prothèse cardiaque (2) de l'alimentation électrique portative (3) et de l'alimentation électrique fixe (4),
- des moyens de gestion (11) construits et agencés pour surveiller et commander l'alimentation électrique (5), et
- une interface de communication (13) avec la prothèse cardiaque (2), **caractérisée en ce que** ladite voie de gestion (10) comporte:
- un isolateur de communication (14) disposé entre les moyens de gestion (11) et l'interface de communication (13).

2. Dispositif de gestion (1) du fonctionnement d'une prothèse cardiaque (2) selon l'une quelconque des revendications précédentes, selon lequel les premiers moyens d'isolation (12) sont conformes à la norme NF-EN 60601-1.

3. Dispositif de gestion (1) du fonctionnement d'une prothèse cardiaque (2) selon l'une quelconque des revendications précédentes, selon lequel l'interface de communication (13) est formée par une interface de type CAN ou de type RS232.

4. Dispositif de gestion (1) du fonctionnement d'une prothèse cardiaque (2) selon l'une quelconque des revendications précédentes, selon lequel la voie de gestion (10) comporte des moyens de mesure (15) construits et agencés pour mesurer une pression atmosphérique.

5. Dispositif de gestion (1) du fonctionnement d'une prothèse cardiaque (2) selon l'une quelconque des revendications précédentes, selon lequel la voie de gestion (10) comporte des moyens d'avertissement (16) construits et agencés pour communiquer avec les moyens de gestion (11) de sorte à avertir un utilisateur lorsqu'un défaut de fonctionnement de l'alimentation électrique (5), de la voie de gestion (10), d'une deuxième voie de gestion (20) et/ou de la prothèse cardiaque (2) est détecté par les moyens de gestion (11).

6. Dispositif de gestion (1) du fonctionnement d'une prothèse cardiaque (2) selon l'une quelconque des revendications précédentes, selon lequel la voie de gestion (10) comporte au moins une interface externe (17) construite et agencée pour permettre des communications entre les moyens de gestion (11) et un dispositif externe.

7. Dispositif de gestion (1) du fonctionnement d'une prothèse cardiaque (2), ledit dispositif de gestion (1) du fonctionnement d'une prothèse cardiaque (2) comportant en outre une deuxième voie de gestion (20), ladite deuxième voie de gestion (20) étant conforme à la voie de gestion (10) selon l'une quelconque des revendications précédentes.

8. Dispositif de gestion (1) du fonctionnement d'une prothèse cardiaque (2) selon la revendication précédente, selon lequel la voie de gestion (10) et la deuxième voie de gestion (20) sont montées en parallèle, ledit dispositif de gestion (1) du fonctionnement comportant en outre des deuxièmes moyens d'isolation (18) de la voie de gestion (10) et de la deuxième voie de gestion (20).

## Patentansprüche

1. Vorrichtung (1) zur Steuerung einer Herzprothese (2), wobei die Vorrichtung (1) zur Steuerung einer Herzprothese (2) einen Steuerungspfad (10) umfasst, wobei der Steuerungspfad (10) Folgendes umfasst:
- eine durch eine tragbare Stromversorgung (3) und eine feste Stromversorgung (4) gebildete Stromversorgung (5),
- Steuerungsmittel (11), konstruiert und angeordnet, um die Stromversorgung (5) einer Herzprothese (2) zu überwachen und zu kontrollieren,
- erste Isoliermittel (12), konstruiert und angeordnet, um die Herzprothese (2) von der tragbaren Stromversorgung (3) und der festen Stromversorgung (4) elektrisch zu isolieren,
- Steuerungsmittel (11), konstruiert und angeordnet, um die Stromversorgung (5) zu überwachen und zu kontrollieren, und
- eine Kommunikationsschnittstelle (13) mit der Herzprothese (2), **dadurch gekennzeichnet, dass** der Steuerungspfad (10) Folgendes umfasst:
- einen Kommunikationsisolator (14), angeordnet zwischen den Steuerungsmitteln (11) und der Kommunikationsschnittstelle (13).

2. Vorrichtung (1) zur Steuerung einer Herzprothese (2) nach einem der vorstehenden Ansprüche, wobei die ersten Isoliermittel (12) der Norm NF-EN 60601-1 entsprechen.

3. Vorrichtung (1) zur Steuerung einer Herzprothese (2) nach einem der vorstehenden Ansprüche, wobei die Kommunikationsschnittstelle (13) durch eine Schnittstelle des CAN-Typs oder RS232-Typs gebildet ist.

4. Vorrichtung (1) zur Steuerung einer Herzprothese (2) nach einem der vorstehenden Ansprüche, wobei der Steuerungspfad (10) Messmittel (15) umfasst, konstruiert und angeordnet, um einen Luftdruck zu messen.

5. Vorrichtung (1) zur Steuerung einer Herzprothese (2) nach einem der vorstehenden Ansprüche, wobei der Steuerungspfad (10) Warnmittel (16) umfasst, konstruiert und angeordnet, um mit den Steuerungsmitteln (11) so zu kommunizieren, dass ein Anwender gewarnt wird, wenn ein Betriebsfehler der Stromversorgung (5), des Steuerungspfads (10), eines zweiten Steuerungspfads (20) und/oder der Herzprothese (2) durch die Steuerungsmittel (11) detektiert wird.

6. Vorrichtung (1) zur Steuerung einer Herzprothese (2) nach einem der vorstehenden Ansprüche, wobei der Steuerungspfad (10) mindestens eine externe Schnittstelle (17) umfasst, konstruiert und angeordnet, um Kommunikationen zwischen den Steuerungsmitteln (11) und einer externen Vorrichtung zu erlauben.

7. Vorrichtung (1) zur Steuerung einer Herzprothese (2), wobei die Vorrichtung (1) zur Steuerung einer Herzprothese (2) des Weiteren einen zweiten Steuerungspfad (20) umfasst, wobei der zweite Steuerungspfad (20) dem Steuerungspfad (10) nach einem der vorstehenden Ansprüche entspricht.

8. Vorrichtung (1) zur Steuerung einer Herzprothese (2) nach einem der vorstehenden Ansprüche, wobei der Steuerungspfad (10) und der zweite Steuerungspfad (20) parallel montiert sind, wobei die Vorrichtung (1) zur Steuerung des Weiteren zweite Isoliermittel (18) des Steuerungspfads (10) und des zweiten Steuerungspfads (20) umfasst.

## Claims

1. A device for managing (1) the operation of a cardiac prosthesis (2), said device for managing (1) the operation of a cardiac prosthesis (2) comprising a management channel (10), said management channel (10) comprising:
- an electrical power supply (5) formed by a portable electrical power supply (3) and a fixed electrical power supply (4),
- management means (11) constructed and arranged to monitor and control the electrical power supply (5) of a cardiac prosthesis (2),
- first isolation means (12) constructed and arranged to electrically isolate the cardiac prosthesis (2) from the portable electrical power supply (3) and from the fixed electrical power supply (4),
- management means (11) constructed and arranged to monitor and control the electrical power supply (5), and
- a communication interface (13) with the cardiac prosthesis (2), **characterized in that** said management channel (10) comprises:
- a communication isolator (14) disposed between the management means (11) and the communication interface (13).

2. The device for managing (1) the operation of a cardiac prosthesis (2) according to any one of the preceding claims, according to which the first isolation means (12) are in compliance with the standard NF-EN 60601-1.

3. The device for managing (1) the operation of a cardiac prosthesis (2) according to any one of the preceding claims, according to which the communication interface (13) is formed by a CAN or RS232 type interface.

4. The device for managing (1) the operation of a cardiac prosthesis (2) according to any one of the preceding claims, according to which the management channel (10) comprises measurement means (15) constructed and arranged to measure atmospheric pressure.

5. The device for managing (1) the operation of a cardiac prosthesis (2) according to any one of the preceding claims, according to which the management channel (10) comprises warning means (16) constructed and arranged to communicate with the management means (11) so as to warn a user when an operational defect of the electrical power supply (5), of the management channel (10), of a second management channel (20) and/or of the cardiac prosthesis (2) is detected by the management means (11).

6. The device for managing (1) the operation of a cardiac prosthesis (2) according to any one of the preceding claims, according to which the management channel (10) comprises at least one external interface (17) constructed and arranged to allow communications between the management means (11) and an external device.

7. The device for managing (1) the operation of a cardiac prosthesis (2), said device for managing (1) the operation of a cardiac prosthesis (2) further comprising a second management channel (20), said second management channel (20) being in accordance with the management channel (10) according to any one of the preceding claims.

8. The device for managing (1) the operation of a cardiac prosthesis (2) according to the preceding claim, according to which the management channel (10) and the second management channel (20) are mounted in parallel, said device for managing (1) the operation further comprising second means of isolating (18) the management channel (10) and the second management channel (20).
